(19) Europäisches Patentamt
European Patent Office
Office européen des brevets

(11) Veröffentlichungsnummer: **0 095 454**
**A2**

(12) # EUROPÄISCHE PATENTANMELDUNG

(21) Anmeldenummer: 83890068.6

(22) Anmeldetag: 02.05.83

(51) Int. Cl.$^3$: C 07 D 317/64
C 07 D 317/46, C 07 D 405/12
C 07 D 407/12, A 61 K 31/36

(30) Priorität: 13.05.82 AT 1888/82
23.12.82 AT 4671/82
12.04.83 AT 1298/83

(43) Veröffentlichungstag der Anmeldung:
30.11.83 Patentblatt 83/48

(84) Benannte Vertragsstaaten:
BE CH DE FR GB IT LI LU NL SE

(71) Anmelder: Gerot-Pharmazeutika Gesellschaft m.b.H.
Arnethgasse 3
A-1171 Wien(AT)

(72) Erfinder: Schlager, Ludwig H., Dr.
Nottebohmstrasse 12
A-1190 Wien(AT)

(74) Vertreter: Pawloy, Heinrich, Dr. et al,
Riemergasse 14
A-1010 Wien(AT)

(54) **Neue kernsubstituierte Pyrogallol-Derivate.**

(57) Die Erfindung sieht neue Pyrogallol-Derivate der allgemeinen Formel (I)

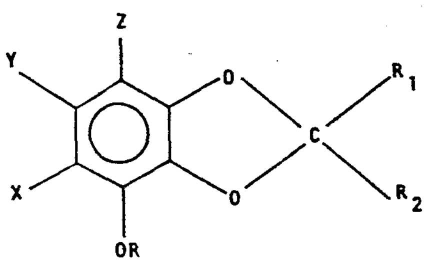

, (I)

worin $R_1$ und $R_2$ gleich oder verschieden sein können und Wasserstoff oder nied. Alkyl bedeuten, und R

(i) Wasserstoff oder

$R_3$
|
(ii) $-C-(CH_2)_n-R_5$ darstellt, wobei
|
$R_4$

(i) wenn R Wasserstoff bedeutet, X, Y und Z Wasserstoff, Halogen oder Nitro bedeuten, mit der Maßgabe, daß nicht alle drei gleichzeitig Wasserstoff darstellen, und mit der weiteren Maßgabe, daß, wenn $R_1$, $R_2$, X und Z Wasserstoff bedeuten, Y keine $NO_2$-Gruppe sein kann; und

$R_3$
|
(ii) a) wenn R die Gruppe $-C-(CH_2)_n-R_5$
|
$R_4$

bedeutet, $R_3$ und $R_4$ gleich oder verschieden sind und Wasserstoff oder nied. Alkyl bedeuten, X, Y und Z ebenfalls gleich oder verschieden sind und Wasserstoff, Halogen oder Nitro bedeuten, n für Null, 1 oder 2 steht, $R_5$ Wasserstoff, Oxiranyl, Mono- oder Dihydroxyalkyl, N-(Dihydroxyalkyl)-amino-hydroxyalkyl, Carboxy, Carbalkoxy, Carbamyl, N-Alkylcarbamyl, N,N-Dialkylcarbamyl, Cyan, $\Delta^2$-Imidazolin-2-yl, Amidoxim, Oximester oder Dialkylacetal darstellt, wobei der Aminrest der Carbamylgruppe auch ein gesättigter Heteroring mit insgesamt zwei Heteroatomen sein kann; oder

b) X Wasserstoff oder Allyl ist, Y, Z, $R_3$ und $R_4$ Wasserstoff sind und n Null ist, $R_5$ die Gruppe

./...

EP 0 095 454 A2

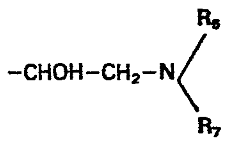

bedeutet, worin $R_6$ Wasserstoff oder nied. Alkyl sein kann oder zusammen mit $R_7$ einen fünf- oder sechsgliedrigen gesättigten heterocyclischen Ring bildet, der höchstens zwei Heteroatome enthält, $R_7$ Alkyl, Alkenyl, Alkinyl, Alkoxyalkyl, Cycloalkyl, Dialkylaminoalkyl, Aryl, Aralkyl, Heteroalkyl, Carbamoylalkyl oder einen fünf- oder sechs-gliedrigen und gegebenenfalls kondensierten heterocyclischen Rest mit höchstens drei Heteroatomen bedeutet,

sowie deren Salze mit anorganischen oder organischen Säuren oder Basen und Verfahren zu deren Herstellung vor.

Diese Verbindungen sind als Agrochemikalien oder als Rohstoffe zu deren Herstellung einerseits und als Rohstoffe zur Herstellung von Arzneimitteln, Röntgenkontrastmitteln oder Fotosensibilisatoren andererseits verwendbar. Insbesondere sind einige der Verbindungen der Formel (I) in Form von Salzen mit Ionenaustauschern als ß-Blocker und Analgetika wertvoll.

# Neue kernsubstituierte Pyrogallol-Derivate

Die Erfindung betrifft neue kernsubstituierte Pyrogallol-Derivate der allgemeinen Formel

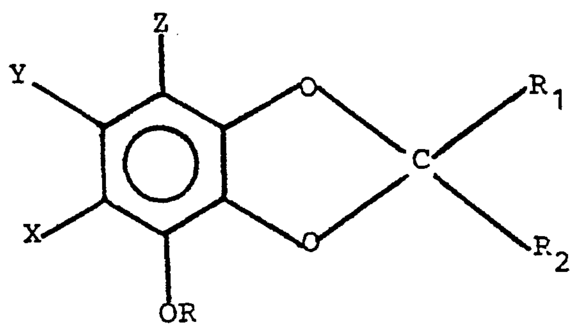

, (I)

worin $R_1$ und $R_2$ gleich oder verschieden sein können und Wasserstoff oder nied.Alkyl bedeuten, und R

(i) Wasserstoff oder

(ii) 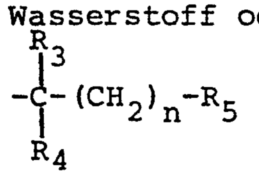 darstellt, wobei

(i) wenn R Wasserstoff bedeutet, X, Y und Z Wasserstoff, Halogen oder Nitro bedeuten, mit der Maßgabe, daß nicht alle drei gleichzeitig Wasserstoff darstellen, und mit der weiteren Maßgabe, daß, wenn $R_1$, $R_2$, X und Z Wasserstoff bedeuten, Y keine $NO_2$-Gruppe sein kann; und

(ii) a) wenn R die Gruppe 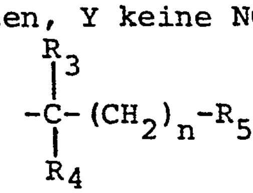 bedeutet, $R_3$ und $R_4$ gleich oder verschieden sind und Wasserstoff oder nied.Alkyl bedeuten, X, Y und Z ebenfalls gleich oder verschieden sind und Wasserstoff, Halogen oder

Nitro bedeuten, n für Null, 1 oder 2 steht, $R_5$ Wasserstoff, Oxiranyl, Mono- oder Dihydroxyalkyl, N-(Dihydroxyalkyl)-amino-hydroxyalkyl, Carboxy, Carbalkoxy, Carbamyl, N-Alkylcarbamyl, N,N-Dialkylcarbamyl, Cyan, $\Delta^2$-Imidazolin-2-yl, Amidoxim, Oximester oder Dialkylacetal darstellt, wobei der Aminrest der Carbamylgruppe auch ein gesättigter Heteroring mit insgesamt zwei Heteroatomen sein kann; oder

b) X Wasserstoff oder Allyl ist, Y, Z, $R_3$ und $R_4$ Wasserstoff sind und n Null ist, $R_5$ die Gruppe 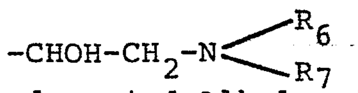 bedeutet, worin $R_6$ Wasserstoff oder nied.Alkyl sein kann oder zusammen mit $R_7$ einen fünf- oder sechsgliedrigen gesättigten heterocyclischen Ring bildet, der höchstens zwei Heteroatome enthält, $R_7$ Alkyl, Alkenyl, Alkinyl, Alkoxyalkyl, Cycloalkyl, Dialkylaminoalkyl, Aryl, Aralkyl, Heteroalkyl, Carbamoylalkyl oder einen fünf- oder sechsgliedrigen und gegebenenfalls kondensierten heterocyclischen Rest mit höchstens drei Heteroatomen bedeutet;

sowie deren Salze mit anorganischen oder organischen Säuren oder Basen.

Weiterhin bezieht sich die Erfindung auf Verfahren zu deren Herstellung.

Die neuen Verbindungen können erfindungsgemäß wie folgt hergestellt werden:

a) Verbindungen der Formel

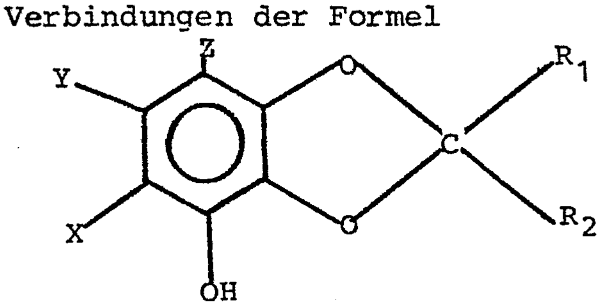

, (Ia)

worin $R_1$,$R_2$, X, Y und Z die in unter (i) angegebene Bedeutung haben, werden dadurch erhalten, daß man ein 4-Hydroxy-1,3-benzodioxol der allgemeinen Formel

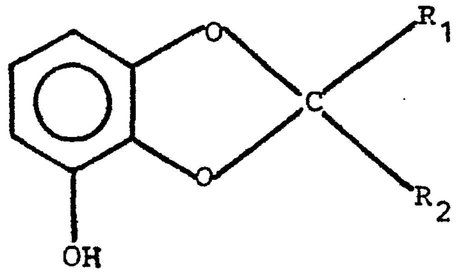

, (II)

worin $R_1$ und $R_2$ die obige Bedeutung haben, vorzugweise in einem inerten Lösungsmittel oder Lösungsmittelgemisch, in ein- oder zweistufiger Reaktion, gegebenenfalls unter Verwendung von säurebindenden Zusätzen und/oder Katalysatoren, mit Halogenierungs- und/oder Nitrierungsmitteln behandelt.

b) Pyrogallroläther der allgemeinen Formel

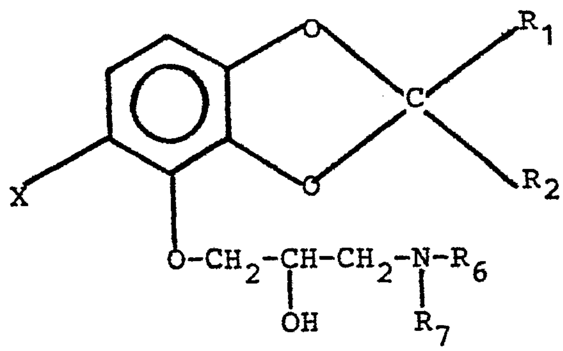

, (Ib)

worin X, $R_1$, $R_2$, $R_6$ und $R_7$ die unter (ii) b) angegebene Bedeutung haben, sowie deren Salze und optisch aktive Isomere werden dadurch erhalten, daß man ein 4-Hydroxy-1,3-benzodioxol der allgemeinen Formel

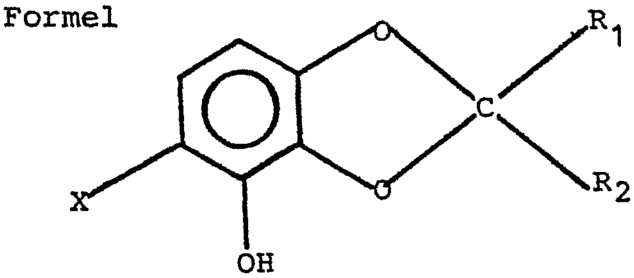

, (III)

worin $R_1$, $R_2$ und X die obige Bedeutung haben, oder ein Alkalisalz hievon mit einem Epihalohydrin umsetzt, das dabei erhaltene Produkt der allgemeinen Formel

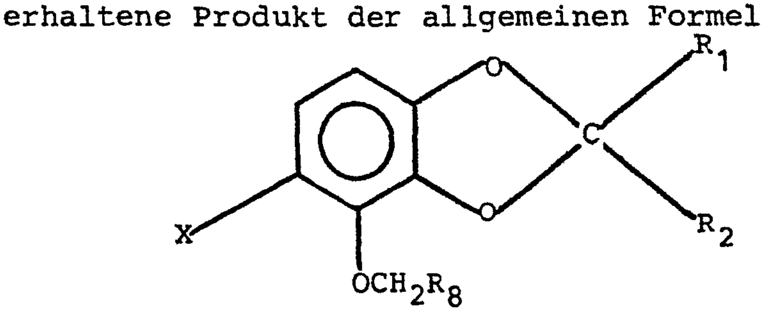

, (IV)

worin $R_8$ $-CHOH.CH_2Q$ oder 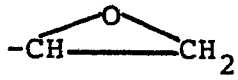 bedeutet, wobei Q ein Halogenatom darstellt, mit einem Amin der allgemeinen Formel

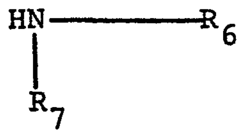

, (V)

worin $R_6$ und $R_7$ die oben angegebene Bedeutung haben, behandelt und das dabei erhaltene Derivat der allgemeinen Formel (Ib) gewünschtenfalls in die optischen Antipoden spaltet und/oder gegebenenfalls mit einer physiologisch verträglichen Säure oder Base in ein Salz überführt.

c) Derivate von Pyrogallol-äthern der allgemeinen Formel

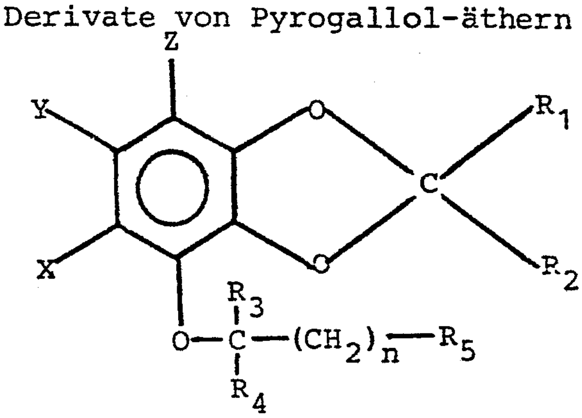

, (Ic)

worin $R_1$, $R_2$, $R_3$, $R_4$, $R_5$, X, Y, Z und n die unter (ii) a) angegebene Bedeutung haben, werden dadurch erhalten, daß man ein Pyrogallol-Derivat der allgemeinen Formel

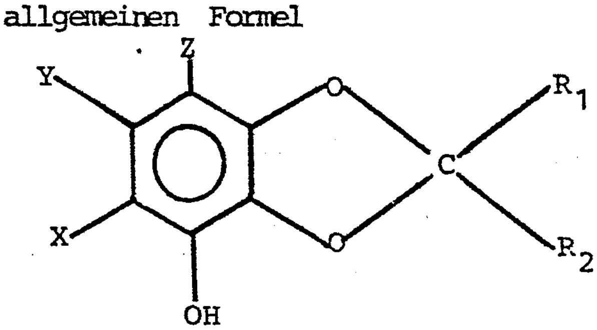

, (VI)

worin $R_1$, $R_2$, X, Y und Z die obige Bedeutung haben, oder ein Phenolat hievon, vorzugsweise in inerten Lösungsmitteln oder Lösungsmittelgemischen, mit einer Verbindung der allgemeinen Formel

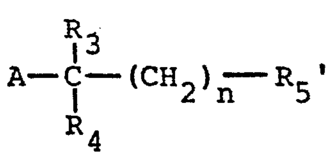

, (VII)

worin $R_3$, $R_4$ und n obige Bedeutung haben, $R_5$' die gleiche Bedeutung wie $R_5$ hat oder eine CH.CN-Gruppe bedeutet und A Halogen, einen Sulfonyloxyrest oder für den Fall, daß $R_3$ und $R_4$ Wasserstoff, n = Null und $R_5$' eine CH.CN-Gruppe darstellen, eine Doppelbindung mit dem benachbarten Kohlenstoffatom bedeutet, umsetzt, gegebenenfalls in einem erhaltenen Derivat der allgemeinen Formel (Ic) eine Gruppe $R_5$ in eine andere Gruppe $R_5$ überführt und, für den Fall, daß X und/oder Y und/oder Z Wasserstoff bedeuten, die erhaltene Verbindung der allgemeinen Formel (Ic) gewünschtenfalls mit Halogenierungs- und/oder Nitrierungsmitteln behandelt.

Einige der erfindungsgemäßen neuen Verbindungen, insbesondere der Formel (Ia) und (Ic) sind einerseits wertvolle Rohstoffe zur Herstellung von Agrochemikalien oder stellen selbst solche dar, andererseits können sie - nach hydrolytischer Aufspaltung des Dioxolanringes - dazu dienen, ansonsten schwer zugängliche kernsubstituierte Pyrogallol-Derivate zu gewinnen. Sie können zur Herstellung von Arzneimitteln, Röntgenkontrastmitteln und Fotosensibilisatoren verwendet werden.

Die neuen Verbindungen der Formel (Ib) liegen vorteilhaft in Form ihrer Salze mit Ionenaustauschern vor.

Diese neuen Pyrogallol-äther der Formel (Ib) sowie deren Salze und optisch aktive Isomere können als ß-blockierende und analgetische Heilmittel Verwendung finden. Bevorzugt ist die Verwendung solcher Heilmittel in Form von Salzen mit Ionenaustauschern.

Unter dem Ausdruck "nied.Alkyl" werden insbesondere verstanden Methyl, Äthyl, Propyl, Butyl, Pentyl, Hexyl, Heptyl und Octyl sowie verzweigte Reste, wie Isoprcpyl, tert.Butyl und 2,2-Dimethylpropyl.

Als Heteroatome für die Ringbildung mit 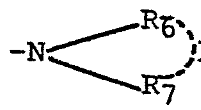 kommen in Frage: Sauerstoff, Stickstoff oder Schwefel.

Als salzbildende Komponenten können entweder physiologisch verträgliche Säuren, wie z.B. N-Cyclohexylsulfaminsäure, Chlorwasserstoffsäure und dgl. Verwendung finden oder optisch aktive Säuren, welche eine Racemattrennung ermöglichen, wie etwa (+)-Campher-10-sulfonsäure(ß), Dibenzoyl-D-weinsäure u.ä. oder aber saure Kationenaustauscher mit vorzugsweise makroporöser Struktur, welche mit den Derivaten der allgemeinen Formel (I) Resinate bilden, aus denen der Wirkstoff nach Applikation retardiert freigesetzt wird. Wenn der Rest $R_6$ eine saure Gruppe (wie z.B. -COOH) enthält, kann auch eine anorganische oder organische Base als salzbildende Komponente dienen, wie z.B. Natriumhydroxyd, Dimethylaminoäthanol, N-Methyl-glucamin und dgl.

Natürlich vorkommende und synthetische Pyrogallol-äther und deren Derivate haben verschiedenartige pharmakologische Wirkungen (Arzneim.-Forsch. 13, 226 (1963)). Das

trifft auch auf solche Verbindungen zu, bei welchen zwei ortho-Sauerstoffatome zu einem 1,3-Benzodioxol-Ring verbunden sind, wie z.B. bei dem in Gewürzölen vorkommenden Apiol oder Myristicin, wovon ersteres antipyretisch und wehenerregend wirkt, während letzterem eine psychotrope Wirkung zugeschrieben wird. Ein Pyrogallol-äther, der im Äther-Rest Stickstoff enthält, das "Gallamin", ist ein Ganglienblocker und Muskelrelaxans.

Basische Pyrogallol-äther der allgemeinen Formel (Ib) sind mit Ausnahme eines Zwischenproduktes (C.Casagrande et al., Boll.Chim.Farm. 112, 445 (1973)) bisher nicht bekannt. Die erfindungsgemäßen neuen Pyrogallol-äther erwiesen sich als ß-Adrenozeptor-blockierende Substanzen, die zum Unterschied von bekannten ß-blockierenden Phenoxy-isopropanolamin-Derivaten überraschenderweise auch stark analgetisch wirken.

Besonders in Anbetracht des Einsatzes von ß-Blockern bei Herzinfarkt, Migräne und Glaukom kommt einer gleichzeitigen analgetischen Wirkung erhöhte Bedeutung zu.

So bewirkt z.B. das Hydrochlorid von 2,2-Dimethyl-4-(2'-hydroxy-3'-tert.butylaminopropoxy)-1,3-benzodioxol am wachen Hund eine 40-fach intensivere Abschwächung der positiv chronotropen Isoprenalinwirkung als der bekannte ß-Blocker Atenolol in gleicher Dosierung und ergibt im Writhing-Test nach Huntingdon mit einer ED 50 von nur 8 mg/kg (Maus, oral) eine gute analgetische Wirkung. Vergleicht man diese im Writhing-Test mit der von bekannten Analgetika, dann zeigt sich die Überlegenheit des neuen Wirkstoffes:

| | ED 50 (mg/kg, Maus, oral) |
|---|---|
| Natriumsalz der Salicylamid-O-essigsäure...... | 11,5 |
| Pethidin-Hydrochlorid ........................ | 13,3 |
| Metamizol-Natrium ............................ | 10,6 |

Die Ausgangsprodukte der allgemeinen Formel (II) sind entweder bekannt oder können nach an sich bekannten Methoden gewonnen werden. Eine Verbindung der allgemeinen Formel (II), worin $R_1$ und $R_2$ Methyl bedeuten, wird in großen Mengen als Zwischenprodukt zur Herstellung von Insektiziden verwendet (Chem.Abstr. 71, 38941m (1969); 78 72110u (1973); 84, 105250u (1976); 85, 192700c (1976); 86, 51598j (1977)).

Obwohl die Bedeutung einer Kernsubstitution mit Halogen- oder Nitrogruppen im Bereich der Agrochemikalien bekannt ist (vgl.: "Wirkstoffe in Pflanzenschutz- und Schädlingsbekämpfungsmitteln", 1. Aufl. 1982, Industrieverband Pflanzenschutz e.V.), und kernhalogenierte Folgeprodukte von Verbindungen der allgemeinen Formel (Ia) in Patenten beansprucht aber nicht beschrieben sind (AT-PSen 283 813, 296 983), erscheinen kernsubstituierte Derivate der allgemeinen Formel (Ia) außer einem durch Nitrierung von Myristicinaldehyd erhaltenen Produkt (J.Chem.Soc. 95, 1161 (1909)) bisher nicht in der Fachliteratur.

Nach dem Verfahren zur Herstellung von Verbindungen (Ia) kann ein 4-Hydroxy-1,3-benzodioxol entweder nur halogeniert oder nitriert oder zuerst halogeniert und anschließend nitriert bzw. zuerst nitriert und danach halogeniert werden. Ein zunächst eingeführter Kernsubstituent kann auch in der darauf folgenden Stufe durch einen anderen ersetzt werden. Bei den Mitteln , die zur Halogenierung bzw. Nitrierung verwendet werden, handelt es sich um solche, die zur Umsetzung mit aromatischen Kohlenwasserstoffen oder Phenolen an sich bekannt sind.

Kernsubstituierte Pyrogallol-Derivate der allgemeinen Formel (Ia) können auch als Zwischenprodukte zur Synthese anderer pharmakologisch wirksamer Verbindungen der Formel (I) dienen oder zur Gewinnung von Fotosensibilisatoren (vgl. GB-PS 1 109 305). Im Kern jodierte Substanzen der allgemeinen Formel (Ia) sind z.B. zur Herstellung von Röntgenkontrastmitteln verwendbar.

Die Möglichkeit des Einsatzes derartiger Derivate in der Humanmedizin wird durch die Erkenntnis gefördert, daß der Heteroring im Human-Test nicht aufgespalten wird (Pestic. Sci. 12, 638, 645 (1981)).

Durch die folgenden Beispiele soll die Erfindung näher erläutert, aber nicht auf diese beschränkt werden. Temperaturangaben beziehen sich jeweils auf Celsiusgrade.

B e i s p i e l 1 :

Zu einer bei 0° gerührten Suspension von 10 g 2,2-Dimethyl-4-hydroxy-1,3-benzodioxol und 22 g $NaHCO_3$ in 150 ml Tetrachlorkohlenstoff tropft man langsam 19,24 g Brom. Die erfolgte Umsetzung ist am Dünnschicht-Chromatogramm (Schicht : Kieselgel 60 F 254, Laufmittel : Chloroform/Metahanol = 9 : 1) zu erkennen. Nach Filtration der Mischung wird das Filtrat am Rotationsverdampfer eingedampft. Der Rückstand kristallisiert beim Stehen und ist aus Petroläther umzukristallisieren. Das erhaltene 2,2-Dimethyl-4-hydroxy-5,7-dibrom-1,3-benzodioxol schmilzt bei 59 - 61°.

Das Natriumsalz dieser Verbindung wird erhalten, wenn man eine methanolische Lösung mit einem Äquivalent einer 30 %-igen Lösung von Natrium-methylat in Methanol versetzt, die Mischung eindampft und den festen Rückstand mit Isopropyläther behandelt. Das getrocknete Natriumsalz schmilzt bis 320° nicht, sondern färbt sich nur braun.

B e i s p i e l 2:

Eine auf -5° gekühlte Lösung von 30 g 2,2-Dimethyl-4-hydroxy-1,3-benzodioxol in 600 ml Chloroform wird unter Rühren tropfenweise mit 14,8 ml einer 65 %igen Salpetersäure (D: 1,40) versetzt, wobei die Temperatur bis auf +2° ansteigt. Am Dünnschicht-Chromatogramm (Schicht: Kieselgel 60 F 254, Laufmittel : Chloroform/Methanol = 9 : 1) sind dann zwei neue Produkte zu erkennen, wovon eines oberhalb

(5-Nitro-Derivat) und eines knapp unterhalb (7-Nitro-Derivat) vom Ausgangspunkt erscheint. Man läßt bei 10° ausreagieren und schüttelt dann dreimal mit Wasser aus, wobei beginnende Kristallisation festzustellen ist. Beim Einengen der mit Aktivkohle und Celite erwärmten und filtrierten Lösung kristallisiert das schwerer lösliche 2,2-Dimethyl-4-hydroxy-7-nitro-1,3-benzodioxol aus. Dieses schmilzt nach dem Umkristallisieren aus $CHCl_3$ bei 193 - 195°.

Zur Gewinnung des 5-Nitro-Derivates wird die $CHCl_3$-Mutterlauge zur Trockene eingedampft und der Rückstand in Isopropanol aufgenommen. Beim Stehen der Lösung in der Kälte kristallisiert das 2,2-Dimethyl-4-hydroxy-5-nitro-1,3-benzodioxol aus, das bei 150 - 151° schmilzt.

Die Stellung der Nitrogruppen ergibt sich aus den NMR-Spektren der beiden Isomeren: Während die OH-Gruppe des 5-Nitro-Derivates in $CDCl_3$ bei 250 MHz ein Signal bei 10,42 ppm zeigt, ergibt die OH-Gruppe der 7-Nitroverbindung unter gleichen Bedingungen eine gleitende Resonanz zwischen 4,6 und 6,2 ppm.

Beide Isomeren liefern beim Erhitzen in wässeriger Lösung infolge Hydrolyse des Dioxolanringes das bekannte 4-Nitropyrogallol, Fp.: 165 - 166° (Literatur: Fp. 162°).

B e i s p i e l 3 :

Eine Lösung von 5 g des gemäß Beispiel 1 erhältlichen Natriumsalzes von 2,2-Dimethyl-4-hydroxy-5,7-dibrom-1,3-benzodioxol in 100 ml Chloroform wird bei 0 bis 5° tropfenweise mit 2,4 ml konz. $HNO_3$ versetzt. Dabei fällt zunächst ein Niederschlag aus, der sich dann weitgehend wieder auflöst. Die orangerote Mischung wird mehrmals mit Wasser ausgeschüttelt, die Chloroform-Phase mit $Na_2SO_4$ getrocknet, nach Verrühren mit Aktivkohle filtriert und im Vakuum eingedampft. Das zurückbleibende Öl kristallisiert beim Verrühren mit Wasser. Nach dem Umkristallisieren des Rohproduktes aus Essigester erhält man das 2,2-Dimethyl-4-hydroxy-5-nitro-7-brom-1,3-benzodioxol als gelbes Pulver mit einem Fp. 93 - 95°.

Die Abspaltung von Bromid während der Umsetzung ist durch Reaktion mit $AgNO_3$ nachzuweisen. Die Stellung der $NO_2$-Gruppe ergibt sich aus dem NMR-Spektrum ($CDCl_3$, 250 MHz), weil die benachbarte OH-Gruppe ein Signal bei 10,27 ppm zeigt (vgl. Beispiel 2).

Beispiel 4:

Zu einer bei 0° gerührten Mischung von 3 g des gemäß Beispiel 2 erhältlichen 2,2-Dimethyl-4-hydroxy-7-nitro-1,3-benzodioxols, 7 g $NaHCO_3$ und 200 ml $CCl_4$ tropft man eine Lösung von 4 g Brom in 20 ml $CCl_4$. Nach dem Erwärmen auf Raumtemperatur läßt man das Gemisch über Nacht stehen. Am Dünnschicht-Chromatogramm ist dann völlige Umsetzung festzustellen. Nach Filtration von anorganischem Material wird das Filtrat im Vakuum eingedampft und der kristalline Rückstand aus wenig Chloroform umkristallisiert. Das erhaltene 2,2-Dimethyl-4-hydroxy-5-brom-7-nitro-1,3-benzodioxol schmilzt bei 185 - 188°, nachdem es bei 155° eine Kristallumwandlung zeigt.

Beim Erhitzen einer wässerigen Lösung dieser Verbindung bildet sich infolge Hydrolyse des Dioxolanringes das gleiche 4-Brom-6-nitro-pyrogallol (Fp. 133 - 135°), das auch durch Hydrolyse des gemäß Beispiel 3 erhältlichen Isomeren entsteht.

Beispiel 5:

Eine Lösung von 10 g 2,2-Dimethyl-4-hydroxy-1,3-benzodioxol in 100 ml Chloroform wird unter Rühren bei 12 bis 14° tropfenweise mit 10 ml 65 %iger $HNO_3$ versetzt, nach 15 Minuten bei gleicher Temperatur mit weiteren 48 ml 65 %iger $HNO_3$. Am Dünnschicht-Chromatogramm (vgl. Beispiel 2) ist ersichtlich, daß die zunächst gebildeten mono-nitrierten Produkte in eines übergehen, das noch unterhalb des 7-Nitro-Derivats erscheint. Man schüttelt die Chloroform-Lösung nach weiteren 15 Minuten Reaktionsdauer dreimal mit Wasser aus, trocknet über $Na_2SO_4$ und dampft das Filtrat im Vakuum ein. Der Rückstand kristallisiert beim Anreiben mit Isopropanol. Aus Äthanol umkristallisiert, schmilzt das erhaltene 2,2-Dimethyl-4-hydroxy-5,7-dinitro-1,3-benzodioxol bei 203 - 205°.

B e i s p i e l 6:

Eine Lösung von 10 g 2,2-Dimethyl-4-hydroxy-1,3-benzodioxol in 200 ml $CCl_4$ wird mit 20 g eines pulverisierten Kationenaustauschers (mit -COONa als aktive Gruppen) versetzt und dazu unter Rühren bei Raumtemperatur eine Lösung von 5,4 ml Sulfurylchlorid in 20 ml $CCl_4$ getropft. Nach 3 Stunden tropft man noch eine Lösung von 2,7 ml $SO_2Cl_2$ in 10 ml $CCl_4$ zu. Nach weiteren 3 Stunden Rühren zeigt das Dünnschicht-Chromatogramm (vgl. Beispiel 1) nahezu vollständige Umsetzung an. Die vom Ionenaustauscher abgesaugte Lösung wird mit Wasser gewaschen, über $Na_2SO_4$ sicc. getrocknet und eingedampft. Durch Umkristallisieren des Rückstandes aus Petroläther unter Verwendung von Aktivkohle erhält man das 2,2-Dimethyl-4-hydroxy-5-chlor-1,3-benzodioxol vom Fp. 81 - 83°.

Die Stellung des Chloratoms ergibt sich daraus, daß die beiden aromatischen Protonen im NMR-Spektrum ($CDCl_3$, 250 MHz) auch nach Bildung des Natriumphenolats in $CD_3OD$ oder eines Komplexes mit einer Europium-Verbindung praktisch keinen "shift"-Effekt zeigen.

B e i s p i e l 7:

Eine Lösung von 5 g 2,2-Dimethyl-4-hydroxy—1,3-benzodioxol in 100 ml $CCl_4$ wird mit einem Jodkristall und 10 g Natriumbicarbonat versetzt und auf 0° gekühlt. Dazu tropft man unter Rühren bei 0 bis 3° eine Lösung von 8,5 g Chlor in 80 ml $CCl_4$. Sobald am Dünnschicht-Chromatogramm (vgl. Beispiel 1) kein Ausgangsprodukt mehr erscheint, entfernt man überschüssiges Chlor durch Einblasen von Stickstoff. Das Filtrat der Reaktionsmischung wird im Vakuum eingedampft und der Rückstand mit Petroläther verrührt, worauf Kristallisation einsetzt. Das Rohprodukt wird unter Verwendung von Aktivkohle aus Methanol/Wasser umgefällt. So erhält man ein farbloses Pulver von 2,2-Dimethyl-4-hydroxy-5,6,7-trichlor-1,3-benzodioxol, das bei 150 - 152° schmilzt.

Im NMR-Spektrum dieser Verbindung erscheinen keine Signale von Kernprotonen.

Beispiel 8:

Zu einer Lösung von 8,4 g 2,2-Dimethyl-4-hydroxy-1,3-benzodioxol in 400 ml 0,5 n NaOH tropft man unter Rühren bei Raumtemperatur eine Lösung von 25,4 g Jod und 30 g Kaliumjodid in 150 ml Wasser. Nach dem Stehen über Nacht ist am Dünnschicht-Chromatogramm (Schicht: Kieselgel 60 F 254, Laufmittel: Äthylacetat/Methanol/2n $NH_4OH$ = 25 : 8 : 3) die Bildung eines neuen Produkts festzustellen. Die filtrierte Lösung wird bei 2 bis 5° mit gesättigter wässeriger $NaHSO_3$-Lösung bis pH 5 verrührt, dann mit Chloroform ausgeschüttelt. Die mit $Na_2SO_4$ getrocknete und nach Zusatz von Aktivkohle filtrierte Chloroformlösung hinterläßt nach dem Eindampfen ein gelbes Öl. Nach dem Umfällen aus Methanol/Wasser oder Umkristallisieren aus Petroläther erhält man 2,2-Dimethyl-4-hydroxy-5,7-dijod-1,3-benzodioxol vom Fp. 52 - 55°.

Beispiel 9:

Eine Mischung von 12,2 g 2,2-Dimethyl-4-hydroxy-1,3-benzodioxol, 24 g $NaHCO_3$, 0,05 g $FeCl_3$ und 400 ml Tetrachlorkohlenstoff wird unter Rühren bei 0° tropfenweise mit einer Lösung von 11 g Chlor in 150 ml $CCl_4$ versetzt. Sobald das Dünnschicht-Chromatogramm (vgl. Beispiel 8) vollständige Umsetzung anzeigt, wird die Mischung filtriert und das Filtrat eingedampft. Der Rückstand wird nach Filtration der Lösung mit Aktivkohle aus Methanol/Wasser umgefällt, dann aus Isopropyläther/Petroläther umkristallisiert. Man erhält so 2,2-Dimethyl-4-hydroxy-5,7-dichlor-1,3-benzodioxol vom Fp. 172-174°.

Die drei kernchlorierten Derivate von Beispiel 6, 7 und 9 lassen sich auch durch ihre Rf-Werte unterscheiden. Unter den Bedingungen des in Beispiel 8 beschriebenen Dünnschicht-Chromatogramms ergibt sich:

| Derivat: | Beispiel: | Rf-Wert: |
|---|---|---|
| 5-Chlor- | 6 | 0,82 |
| 5,7-Dichlor- | 9 | 0,73 |
| 5,6,7-Trichlor- | 7 | 0,67 |

B e i s p i e l  10:

Eine Lösung von 113,5 g 2,2-Dimethyl-4-hydroxy-1,3-benzodioxol in 400 ml Methanol wird unter Rühren tropfenweise mit 123 g einer 30 %igen Lösung von Natriummethylat in Methanol versetzt, wobei sich die Mischung dunkel färbt. Das durch Eindampfen am Rotationsverdampfer bei 50° erhaltene Öl (140g) wird in 180 ml Epichlorhydrin aufgenommen und die Lösung nach Zusatz von 15 g Benzyltriäthyl-ammoniumchlorid 3 Stunden bei 55° gerührt. Am Dünnschicht-Chromatogramm (Fertigplatte: Kieselgel 60 F 254, Laufmittel: Benzol/Dioxan/Eisessig, 90 : 25 : 4 Vol.Teile) ist dann kein Ausgangsprodukt mehr zu erkennen. Überschüssiges Epichlorhydrin wird am Rotationsverdampfer bei 60° abgedampft und der Rückstand in heißem Äthylacetat aufgenommen. Nach Zusatz von etwas Aktivkohle zu der erkalteten Lösung saugt man durch Celite-Filterhilfsmittel ab und dampft das Filtrat im Vakuum ein. Man erhält so 151 g (99,5 %) rohes 2,2-Dimethyl-4-(2',3'-epoxy-propoxy)-1,3-benzodioxol als gelbes Öl, das zur weiteren Umsetzung mit Basen hinreichend rein ist.

10 g rohes 2,2-Dimethyl-4-(2',3'-epoxy-propoxy)-1,3-benzodioxol werden in 20 ml Äthanol nach Zusatz von 10 ml tert. Butylamin 3 Stunden unter Rückfluß erhitzt. Eine Probe zeigt dann am Dünnschicht-Chromatogramm (Fertigplatte: Kieselgel 60 F 254, Laufmittel: Essigester/Methanol/2n $NH_4OH$, 25 : 8 : 3 Vol.Teile) vollständige Umsetzung an. Man dampft die Lösung am Rotationsverdampfer bei 50° ein, nimmt den öligen Rückstand in etwas Essigester auf und dampft nochmals ein. Das erhaltene Öl wird in Essigester gelöst und die Lösung durch Ausschütteln mit gesättigter wässeriger NaCl-Lösung gewaschen. Danach extrahiert man die Essigesterlösung mit 2n HCl, filtriert die saure wässerige Lösung nach Zusatz von Aktivkohle, macht das Filtrat mit 5n NaOH bis pH 10 alkalisch und extrahiert mehrmals mit Chloroform. Die mit $Na_2SO_4$ getrocknete Chloroformlösung hinterläßt nach dem Eindampfen ein Öl, das beim Stehen kristallisiert. Aus n-Hexan umkristallisiert, erhält man 10,5 g (79 % d. Th.) farblose Kristalle von 2,2-Dimethyl-4-(2'-hydroxy-3'-tert.butylamino--propoxy)-1,3-benzodioxol mit Fp. 75 - 76°.

Das Hydrochlorid dieser Verbindung fällt aus einer Isopropyläther-Lösung beim Versetzen mit alkoholischer HCl. Aus Isopropanol umkristallisiert, schmilzt das Hydrochlorid bei 124 - 126°.

B e i s p i e l 11 :

Eine Lösung von 10 g des gemäß Beispiel 10 erhältlichen 1,3-Benzodioxol-Ausgangsprodukts in 20 ml Äthanol wird mit 8,24 g Benzhydrylamin versetzt und 5 Stunden unter Rückfluß gekocht. Der nach Eindampfen der Mischung verbleibende Rückstand wird in etwas Aceton aufgenommen und die Lösung mit alkoholischer HCl sowie mit Isopropyläther versetzt. Der dabei ausfallende ölige Niederschlag wird beim Stehen kristallin. Nach dem Umkristallisieren aus Isopropanol (unter Verwendung von Aktivkohle und Filtration) erhält man 18 g (90,5 %) Hydrochlorid von 2,2-Dimethyl-4-(2'-hydroxy-3'-diphenyl-methylamino-propoxy)-1,3-benzodioxol, das bei 136 - 139° schmilzt.

B e i s p i e l 12 :

Eine Lösung von 10 g des gemäß Beispiel 10 erhältlichen 1,3-Benzodioxol-Ausgangsprodukts und 4,15 g 2-Methyl-3-butin-2-ylamin (90 %ig) in 20 ml Äthanol läßt man 3 Tage bei Raumtemperatur stehen. Der Eindampfrückstand wird in Diäthyläther gelöst und die Lösung mit alkohol. HCl versetzt, wobei sich ein Öl abscheidet. Dieses wird in heißem Aceton gelöst. Beim Abkühlen kristallisiert das Hydrochlorid von 2,2-Dimethyl-4-[2'-hydroxy-3'-(2"-methyl-3"-butin-2"-ylamino)-propoxy]-1,3-benzodioxol aus. Fp.: 153 - 155°. Ausbeute: 12,5 g (81,3 %).

B e i s p i e l 13:

Eine Lösung von 22,1 g 2-Methoxy-2-methyl-4-hydroxy-1,3-benzodioxol in 60 ml Methanol wird unter Rühren tropfenweise mit 21,8 g einer 30 %igen Lösung von Natriummethylat in Methanol versetzt, wobei sich die Mischung dunkel färbt. Das durch Eindampfen am Rotationsverdampfer bei 50° erhaltene Öl wird

in 50 ml Epichlorhydrin aufgenommen und die Mischung nach Zusatz von 3,5 g Benzyl-triäthylammoniumchlorid 3 Stunden bei 60° gerührt. Man dampft überschüssiges Epichlorhydrin am Rotationsverdampfer bei 60° ab und nimmt den Rückstand in heißem Äthylacetat auf. Nach Zusatz von etwas Aktivkohle zu der erkalteten Lösung saugt man durch Celite-Filterhilfsmittel ab und dampft das Filtrat im Vakuum ein. Man erhält so 28,5 g (98,6 %) rohes 2-Methoxy-2-methyl-4-(2',3'-epoxy-propoxy)-1,3-benzodioxol als braunes Öl, das zur weiteren Umsetzung mit Basen hinreichend rein ist.

Eine Lösung von 10 g 2-Methoxy-2-methyl-4-(2',3'-epoxy-propoxy)-1,3-benzodioxol in 20 ml Äthanol wird nach Zusatz von 10 ml Isopropylamin unter Rückfluß erhitzt, bis eine Probe am Dünnschicht-Chromatogramm (Fertigplatte: Kieselgel 60 F 254, Laufmittel: Chloroform/Methanol, 9 : 1 Vol.Teile oder Essigester/Methanol/2n $NH_4OH$, 25 : 8 : 3 Vol.Teile) vollständige Umsetzung anzeigt. Das nach dem Eindampfen der Lösung am Rotationsverdampfer zurückbleibende rote Öl wird in Aceton gelöst und mit einer Lösung von 8,5 g Cyclohexylsulfaminsäure in Aceton versetzt. Beim Stehen der Mischung bei 4° kristallisiert das Cyclamat von 2-Methoxy-2-methyl-4-(2'-hydroxy-3'-isopropylamino-propoxy)-1,3-benzodioxol, das nach dem Umkristallisieren aus Isopropanol bei 139 - 142° schmilzt. Ausbeute: 12,6 g (63 % d.Th.).

<u>B e i s p i e l 14 :</u>

Eine Lösung von 10 g des gemäß Beispiel 10 erhältlichen 2,2-Dimethyl-4-(2',3'-epoxy-propoxy)-1,3-benzodioxols in 20 ml Äthanol wird nach Zusatz von 10 ml Isopropylamin 3 Stunden unter Rückfluß erhitzt und danach im Vakuum eingedampft. Das so erhaltene gelbe Öl nimmt man in Isopropyläther auf. Beim Versetzen der Lösung mit alkoholischer HCl fällt das Hydrochlorid von 2,2-Dimethyl-4-(2'-hydroxy-3'-isopropylamino-propoxy)-1,3-benzodioxol aus (12,0 g, 83,9 %), das nach dem Umkristallisieren aus Isopropanol bei 157 - 159° schmilzt. Die entsprechende freie Base schmilzt bei 91-93°.

In eine Lösung von 10 g des erhaltenen Hydrochlorids in 140 ml aqua dest. werden unter Rühren portionsweise 20 g eines fein-pulverigen Polystyrol-Kationenaustauschers, der $-SO_3Na$ als aktive Gruppen enthält, eingetragen. Man rührt noch 24 Stunden, saugt ab, wäscht den Niederschlag am Filter mehrmals mit aqua dest. und erhält nach dem Trocknen im Vakuum bei 70° 25,5 g Resinat.

Nach oraler Verabreichung dieses Resinats z.B. in magensaft-resistenten Kapseln wird der Wirkstoff retardiert frei-gesetzt.

B e i s p i e l 15 :

Eine Mischung von 20 g p-Chlorphentermin-Hydrochlorid, 150 ml gesättigter $NaHCO_3$-Lösung und 200 ml Chloroform wurde 1 Stunde intensiv gerührt, dann die Phasen getrennt, die $CHCl_3$-Phase über $Na_2SO_4$ sicc. getrocknet und im Vakuum eingedampft. Die p-Chlorphentermin-Base wurde als gelbliches Öl erhalten.

8,3 g dieses Öls wurden mit 10 g des gemäß Beispiel 10 erhältlichen 2,2-Dimethyl-4-(2',3'-epoxy-propoxy)-1,3-benzodioxols in 20 ml Äthanol 8 Stunden unter Rückfluß erhitzt. Das nach dem Eindampfen der Lösung am Rotationsverdampfer zurückbleibende gelbliche Öl wurde in Aceton aufgenommen und die Lösung mit alkoholischer HCl versetzt. Das dabei ausgefallene Hydrochlorid von 2,2-Dimethyl-4-[3'-(α,α-dimethyl-p-chlorphenäthyl-amino)-2'-hydroxy-propoxy]-1,3-benzodioxol (15,7 g, 78,9 %) wurde aus Äthanol umkristallisiert. Fp. 212 - 215°.

Nach Verfahren, die den beschriebenen Beispielen 10 bis 15 analog sind, wurden weitere Derivate der allgemeinen Formel (Ib) erhalten. Diese sind in den folgenden Tabellen I und II zusammengestellt:

Tabelle I ( X = H)

| $R_1$ | $R_2$ | $R_6$ | $R_7$ | Fp°C (Base/Salz) |
|---|---|---|---|---|
| $CH_3$ | $CH_3$ | H | $-CH_2-C(CH_3)_3$ | 116-118 (Cyclamat) |
| $CH_3$ | $CH_3$ | H | $-(CH_2)_3.CH_3$ | 69-74 (Base) |
| $CH_3$ | $CH_3$ | H | $-(CH_2)_5.CH_3$ | 110-111 (Hydrochlorid) |
| $CH_3$ | $CH_3$ | H | 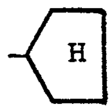 | 114-117 (Hydrochlorid) |
| $CH_3$ | $CH_3$ | H | 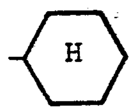 | 153-156 (Hydrochlorid) |
| $CH_3$ | $CH_3$ | $CH_3$ | 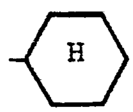 | 57-59 (Base) |
| $CH_3$ | $CH_3$ | H | $-(CH_2)_2-O-CH_3$ | 109-111 (Cyclamat) |
| $CH_3$ | $CH_3$ | H | $-(CH_2)_3-O-CH_3$ | 85-88 (Cyclamat) |
| $CH_3$ | $CH_3$ | H | $-(CH_2)_3-N(C_2H_5)_2$ | 155-157 (Dihydrochlorid) |
| $CH_3$ | $CH_3$ | H | $-(CH_2)_3.CONH_2$ | 110-112 (Cyclamat) |
| $CH_3$ | $CH_3$ | H | $-CH_2$ 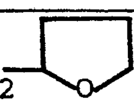 | 116-120 (Hydrochlorid) |
| $CH_3$ | $CH_3$ | H | 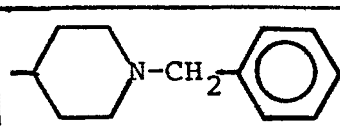 | 246-248 (Dihydrochlorid) |
| $CH_3$ | $CH_3$ | H | 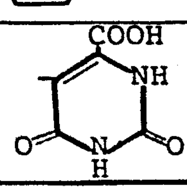 | 211-214 (Base) |

Tabelle I (X = H) Fortsetzung

| $R_1$ | $R_2$ | $R_6$ | $R_7$ | Fp°C (Base/Salz) |
|---|---|---|---|---|
| $CH_3$ | $O-CH_3$ | H | $-C(CH_3)_3$ | 160-162 (Cyclamat) |
| $CH_3$ | $O-CH_3$ | H | 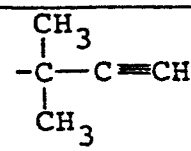 | 108-112 (Base) |
| $CH_3$ | $CH_3$ | H | 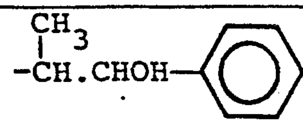 | 111-112 (Base) |
| $CH_3$ | $CH_3$ | H | 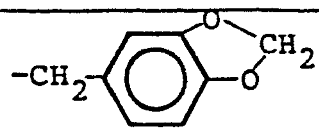 | 145-146 (Base) |
| $CH_3$ | $CH_3$ | H | 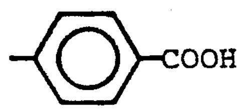 | 102-107 (Hydrochlorid) |
| $CH_3$ | $CH_3$ | H | $-CH_2-CH=CH_2$ | 115-117 (Hydrochlorid) |
| $CH_3$ | $CH_3$ | H | 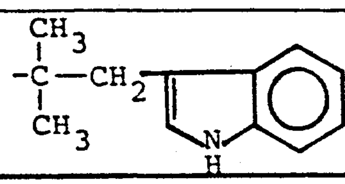 | 121-123 (Hydrochlorid) |

Tabelle II ( X = H)

| $R_1$ | $R_2$ | 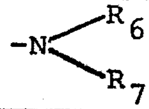 | Fp°C (Base/Salz) |
|---|---|---|---|
| $CH_3$ | $CH_3$ | 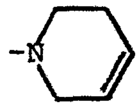 | 131-133 (Cyclamat) |
| $CH_3$ | $CH_3$ | 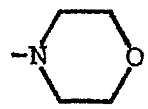 | 135-137 (Cyclamat) |
| $CH_3$ | $CH_3$ | 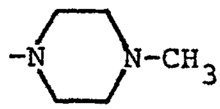 | 188-195 (Dihydrochlorid) |
| $CH_3$ | $CH_3$ | 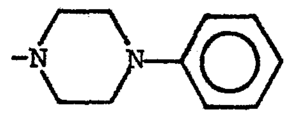 | 177-179 (Hydrochlorid) |
| $CH_3$ | $CH_3$ | 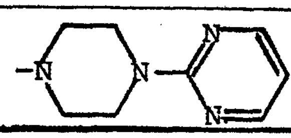 | 174-176 (Hydrochlorid) |

B e i s p i e l 16 :

a.) Eine Mischung von 120 g 2,2-Dimethyl-4-hydroxy-1,3-benzodioxol, 319,6 g Allylbromid, 533 g $K_2CO_3$ und 2600 ml Aceton wird 5 Stunden unter Rückfluß gekocht und dann filtriert. Der Eindampfrückstand des Filtrats wird mit Isopropyläther und verdünnter NaOH geschüttelt, die Ätherlösung mit Wasser gewaschen, mit $Na_2SO_4$ sicc. getrocknet und nach Behandlung mit Aktivkohle filtriert. Durch Eindampfen des Filtrats erhält man ein grünlich-gelbes Öl, das bei 122 - 124°/10 Torr siedet und aus 2,2-Dimethyl-4-allyloxy-1,3-benzodioxol besteht. Ausbeute: 132 g (88,6 %).

b.) Man erhitzt eine Lösung von 132 g 2,2-Dimethyl-4-allyloxy-1,3-benzodioxol in 300 g 1,2-Dichlor-benzol 4 Stunden unter Rühren auf 180°, extrahiert die erkaltete Lösung mehrmals mit 1n NaOH, säuert die vereinigten wässerigalkalischen Extrakte unter Kühlung mit verdünnter HCl an und schüttelt sofort mit Chloroform aus. Die getrocknete Chloroformlösung hinterläßt nach dem Eindampfen 105,3 g (79,8 %) eines gelben Öls, das aus 2,2-Dimethyl-4-hydroxy-5-allyl-1,3-benzodioxol besteht. Rf-Wert auf Kieselgel 60 F 254 (Laufmittel: Chloroform/Methanol = 9 : 1) : 0,66. Rf-Wert des zur Allylumlagerung eingesetzten Allyläthers unter gleichen DC-Bedingungen: 0,73.

c.) Zu einer Lösung von 105,3 g 2,2-Dimethyl-4-hydroxy-5-allyl-1,3-benzodioxol in 150 ml Methanol tropft man unter Rühren 91,7 g einer 30 %igen methanolischen Lösung von $CH_3ONa$. Nach 2 Stunden dampft man die Lösung ein, nimmt den dunklen Rückstand in 100 ml Epichlorhydrin unter Zusatz von 11,2 g Benzyltriäthylammoniumchlorid auf, rührt 5 Stunden und läßt die Lösung über Nacht stehen.

Der Eindampfrückstand wird zwischen Wasser und Äthylacetat verteilt. Die Essigesterlösung wird noch mehrmals mit Wasser ausgeschüttelt, getrocknet und eingedampft. Man erhält so 127 g (94,8 %) 2,2-Dimethyl-4-(2',3'-epoxy-propoxy)-5-allyl-1,3-benzodioxol als gelbes Öl, das unter den bei Stufe b.) beschriebenen DC-Bedingungen einen Rf-Wert von 0,74 zeigt.

d.) 20 g 2,2-Dimethyl-4-(2',3'-epoxy-propoxy)-5-allyl-1,3-benzodioxol und 25 ml tert.Butylamin werden in 25 ml Äthanol 2 Tage bei Raumtemperatur gerührt. Der nach dem Eindampfen der Lösung erhaltene Rückstand wird in Isopropyläther aufgenommen und die Lösung mit alkoholischer HCl versetzt, worauf das Hydrochlorid von 2,2-Dimethyl-4-(2'-hydroxy-3'-tert.butylamino-propoxy)-5-allyl-1,3-benzodioxol ausfällt. Nach dem Umkristallisieren aus Isopropyläther/Äthylacetat (3 : 1) schmilzt das Produkt bei 129 - 132°. Ausbeute: 18,9 g (66,6 %).

Entsprechend den in Beispiel 16 gemachten Angaben erhält man die in Tabelle III angeführten Verbindungen.

Tabelle III

$R_1$ = $CH_3$

$R_2$ = $CH_3$

X = $CH_2=CH-CH_2$

| $R_6$ | $R_7$ | Fp°C (Base/Salz) |
|---|---|---|
| H | $-CH(CH_3)_2$ | 120-124 (Hydrochlorid) |
| H | 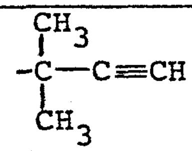 | 152-156 (Hydrochlorid) |
| H | 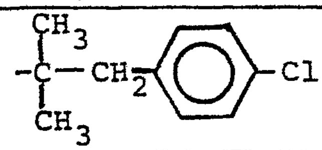 | 160-165 (Hydrochlorid) |
| 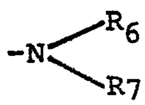 | | |
| 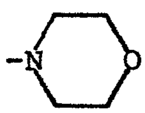 | | 153-158 (Hydrochlorid) |
| 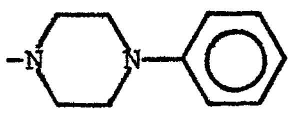 | | 145-152 (Dihydrochlorid) |

B e i s p i e l 17 :

Zu einer Lösung von 10 g 2,2-Dimethyl-4-hydroxy-1,3-benzodioxol in 50 ml Methanol tropft man unter Rühren 10,8 g einer 30 %igen Lösung von $CH_3ONa$ in Methanol. Die dunkel gefärbte Mischung wird dann mit einer Lösung von 12,55 g Bromessigsäureäthylester in 15 ml Methanol tropfenweise versetzt. Man rührt bei Raumtemperatur, bis am Dünnschicht-Chromatogramm (Schicht: Kieselgel 60 F 254, Laufmittel: Chloroform/ Methanol = 9 : 1) kein Ausgangsprodukt mehr erscheint, dampft am Rotationsverdampfer ein und nimmt den Rückstand in Chloroform auf. Die filtrierte Chloroformlösung wird zweimal mit 1n NaOH und dreimal mit Wasser ausgeschüttelt, über $Na_2SO_4$

getrocknet und eingedampft. Das zunächst als Öl erhaltene 2,2-Dimethyl-4-(carbäthoxy-methoxy)-1,3-benzodioxol kristallisiert beim Stehen und schmilzt dann bei 46 - 48°.

Beispiel 18:

Eine Lösung von 11,3 g des gemäß Beispiel 17 erhaltenen Esters in 100 ml Äthanol wird nach Zusatz von 20 ml Wasser unter Rühren tropfenweise mit 8 g einer 30 %igen Lösung von $CH_3ONa$ in Methanol versetzt. Nach einer Stunde Rühren bei Raumtemperatur zeigt das Dünnschicht-Chromatogramm (Schicht: Kieselgel 60 F 254, Laufmittel: Essigester/Methanol/2n $NH_4OH$ = 25 : 8 : 3) vollständige Umsetzung an. Die Lösung wird im Vakuum eingedampft, der Rückstand in Wasser gelöst. Der beim Ansäuern der wässerigen Lösung mit HCl ausfallende Niederschlag wird abgesaugt, mit Wasser gewaschen und im Vakuum getrocknet. Nach dem Umkristallisieren aus $CHCl_3/CCl_4$ erhält man das bei 138 - 141° schmelzende 2,2-Dimethyl-4-(carboxy-methoxy)-1,3-benzodioxol.

Das Natriumsalz dieser Verbindung kann man z.B. durch Umsetzung einer methanolischen Lösung der freien Säure mit einem Äquivalent Natrium-methylat, Eindampfen der Mischung und Behandeln des Rückstandes mit Isopropyläther erhalten; es schmilzt bei 256 - 260° unter Zersetzung.

Beispiel 19:

Eine Mischung von 4 g der gemäß Beispiel 18 erhältlichen Carbonsäure, 8 g $NaHCO_3$ und 100 ml $CCl_4$ wird nach Zusatz eines Jodkristalls unter Rühren bei -5° tropfenweise mit einer Lösung von 4,5 g Chlor in 100 ml $CCl_4$ versetzt. Man läßt bei 0° ausreagieren und filtriert dann. Der erhaltene Niederschlag wird mit $CHCl_3$ heiß extrahiert, der Extrakt mit dem $CCl_4$-Filtrat vereinigt und im Vakuum eingedampft. Den Eindampfrückstand behandelt man mit $CHCl_3/H_2O$, trennt die Phasen und dampft die $CHCl_3$-Phase ein. Der kristalline Rückstand wird aus $CCl_4$ umkristallisiert, wobei man das 2,2-Dimethyl-4-(carboxymethoxy)-5,7-dichlor-1,3-benzodioxol vom Fp. 143 - 145° erhält.

Beispiel 20:

Das Produkt von Beispiel 19 ist auch auf folgendem Wege zugänglich:

2,2-Dimethyl-4-hydroxy-5,7-dichlor-1,3-benzodioxol wird nach dem im Beispiel 17 beschriebenen Verfahren zu 2,2-Dimethyl-4-(carbäthoxy-methoxy)-5,7-dichlor-1,3-benzodioxol umgesetzt, welches bei 82 - 84° schmilzt.

Dieser Ester ergibt nach Verseifung gemäß Beispiel 18 die entsprechende freie Säure, welche den im Beispiel 19 angegebenen Schmelzpunkt zeigt.

Beispiel 21:

Eine Mischung von 6,5 g des gemäß Beispiel 17 erhältlichen Esters, 8 g Morpholin, 6 ml Dimethylformamid und 20 ml Benzol wird unter Rückfluß erhitzt, bis am Dünnschicht-Chromatogramm (vgl. Beispiel 18) kein Ausgangsprodukt mehr erscheint. Der nach dem Eindampfen der Lösung erhaltene Rückstand wird aus Isopropyläther umkristallisiert, besteht aus 2,2-Dimethyl-4-(morpholino-carbomethoxy)-1,3-benzodioxol und schmilzt bei 92 - 93°.

Beispiel 22:

Eine Lösung von 6,3 g 2,2-Dimethyl-4-hydroxy-1,3-benzodioxol in 30 ml Acrylnitril wird mit 2 Tropfen einer 40 %igen methanolischen Lösung von Benzyltrimethylammonium-hydroxyd versetzt und dann unter Rühren auf Rückfluß erhitzt, bis am Dünnschicht-Chromatogramm (vgl. Beispiel 17) nur mehr Spuren von Ausgangsprodukt zu sehen sind. Der Eindampfrückstand wird in Essigester/Wasser aufgenommen, die Mischung filtriert, die Essigester-Phase mit 1 n NaOH und nochmals mit Wasser ausgeschüttelt und die mit $Na_2SO_4$ getrocknete Essigesterlösung eingedampft.

Der ölige Rückstand, 2,2-Dimethyl-4-(2'-cyanäthoxy)-1,3-benzodioxol, wird für die folgende Umsetzung mit Hydroxylamin verwendet.

Dazu versetzt man unter Außenkühlung mit Wasser eine Lösung von 3,5 g Hydroxylamin-Hydrochlorid in 15 ml Methanol unter Rühren tropfenweise mit 9 g einer 30 %igen methanolischen $NaOCH_3$-Lösung und saugt vom ausgefallenen NaCl ab. Die so erhaltene Lösung wird zu einer unter Rückfluß gehaltenen und gerührten Lösung von 6,2 g 2,2-Dimethyl-4-(2'-cyanäthoxy)-1,3-benzodioxol in 25 ml Äthanol getropft. Nach weiteren 15 Minuten Rückfluß ist gemäß Dünnschicht-Chromatogramm (vgl. Beispiel 17) alles umgesetzt. Die filtrierte Lösung wird eingedampft. Der ölige Rückstand kristallisiert aus Isopropanol, besteht aus 2,2-Dimethyl-4-(2'-carbamidoxim-äthoxy)-1,3-benzodioxol und schmilzt bei 100 - 103°.

B e i s p i e l 23:

Die Lösung von 10 g Natriumsalz von 2,2-Dimethyl-4-hydroxy-5,7-dibrom-1,3-benzodioxol und 0,7 g Benzyltriäthylammoniumchlorid in 40 ml Chloracetonitril wird unter Rückfluß gerührt, bis das Dünnschicht-Chromatogramm (vgl. Beispiel 17) vollständige Umsetzung anzeigt. Man dampft im Vakuum ein, nimmt den Rückstand in Essigester auf und schüttelt mit 1n NaOH und danach mit Wasser aus. Der nach dem Eindampfen der Essigesterlösung verbleibende Rückstand kristallisiert beim Stehen im Kühlschrank. Aus Petroläther umkristallisiert schmilzt das erhaltene 2,2-Dimethyl-4-cyanmethoxy-5,7-dibrom-1,3-benzodioxol bei 64 - 66°.

B e i s p i e l 24 :

Eine Lösung von 2,2 g Hydroxylamin-Hydrochlorid in 15 ml Methanol wird unter Rühren bei 12° tropfenweise mit 5,7 g einer 30 %igen methanolischen Lösung von $NaOCH_3$ versetzt. Das Filtrat der erhaltenen Mischung tropft man unter Rühren zu einer unter Rückfluß kochenden Lösung von 7,4 g 2,2-Dimethyl-4-cyanmethoxy-5,7-dibrom-1,3-benzodioxol in 25 ml Äthanol. Etwa 15 Minuten danach wird am Rotationsverdampfer eingedampft. Man behandelt den Rückstand mit Aceton und kristallisiert den entstandenen Niederschlag aus Isopropanol um. Das erhaltene 2,2-Dimethyl-4-(carbamidoxim-methoxy)--5,7-dibrom-1,3-benzodioxol schmilzt bei 153 - 155°.

Beispiel 25:

Eine Lösung von 10 g 2,2-Dimethyl-4-hydroxy-5,7-dijod-1,3-benzodioxol in 50 ml Methanol wird mit 4,3 g einer 30 %igen methanolischen Lösung von $NaOCH_3$ versetzt und danach im Vakuum eingedampft. Der Rückstand wird in 50 ml Äthanol aufgenommen und die Lösung nach Zusatz von 2,65 g 3-Chlor-1,2-propandiol unter Rückfluß erhitzt, bis am Dünnschicht-Chromatogramm (vgl. Beispiel 17) nur mehr Spuren von Ausgangsprodukt erscheinen. Die über Celite filtrierte Lösung wird im Vakuum eingedampft, der Rückstand in $CHCl_3/H_2O$ aufgenommen, die $CHCl_3$-Phase mit $Na_2SO_4$ getrocknet und eingedampft. Der Rückstand kristallisiert aus Isopropanol nach Filtration der heißen Lösung mit Aktivkohle. Das entstandene 2,2-Dimethyl-4-(2',3'-epoxy-propoxy)-5,7-dijod-1,3-benzodioxol schmilzt bei 112 - 114°.

Beispiel 26:

Das Natriumsalz von 5 g 2,2-Dimethyl-4-hydroxy-5,7-dijod-1,3-benzodioxol wird in Methanol durch Zusatz von 2,15 g einer 30 %igen methanolischen $NaOCH_3$-Lösung und Eindampfen der Mischung hergestellt. Der Rückstand wird nach Zusatz von 0,5 g Triäthylbenzylammoniumchlorid in 30 ml Epichlorhydrin aufgenommen und über Nacht stehen gelassen. Der Eindampfrückstand wird dann mit heißem Essigester behandelt und die filtrierte Lösung eingedampft. Man erhält so 5,6 g 2,2-Dimethyl-4-(2',3'-dihydroxy-propoxy)-5,7-dijod-1,3-benzodioxol als gelbes Öl. Am Dünnschicht-Chromatogramm (vgl. Beispiel 18) wandert es etwas höher als das Ausgangsprodukt und erweist sich als rein. Beim Stehen kristallisiert das Produkt und schmilzt dann bei 64 - 67°.

Beispiel 27:

Zu einer Lösung von 1,7 g 2,2-Dimethyl-4-(carboxy-methoxy)-5,7-dijod-1,3-benzodioxol in 25 ml frisch destilliertem Dimethylformamid tropft man unter Rühren 1 ml Thionylchlorid. Am nächsten Tag gibt man eine 33 %-ige Lösung von Methylamin

in absol. Äthanol zu, bis die Mischung alkalisch reagiert und läßt diese nochmals über Nacht stehen. Die Lösung wird im Vakuum eingeengt und dann mit gesättigter Kochsalzlösung gefällt. Der erhaltene Niederschlag wird aus Äthanol/Wasser umkristallisiert. Das getrocknete 2,2-Dimethyl-4-(N-methyl-carbamyl-methoxy)-5,7-dijod-1,3-benzodioxol schmilzt bei 146 - 148°.

B e i s p i e l 28 :

Nach dem Verfahren gemäß Beispiel 23 erhält man aus dem Natriumsalz von 2,2-Dimethyl-4-hydroxy-1,3-benzodioxol das 2,2-Dimethyl-4-cyanmethoxy-1,3-benzodioxol als orangefarbiges Öl.

10 g dieses Öls werden nach Zusatz von 0,1 g Schwefel in 20 ml Äthylendiamin eine Stunde auf 100° erhitzt. Der Eindampfrückstand der Reaktionsmischung wird dann in Essigester aufgenommen, die filtrierte Lösung mehrmals mit Wasser ausgeschüttelt und dann mit 2n HCl extrahiert. Die saure wässerige Phase wird durch NaOH-Zusatz alkalisch gemacht und mit Chloroform ausgeschüttelt. Nach dem Eindampfen der mit $Na_2SO_4$ sicc. getrockneten Chloroformlösung erhält man ein gelbliches Pulver. Aus Isopropanol umkristallisiert, bildet das entstandene 2,2-Dimethyl-4-($\Delta^2$-imidazolin-2-yl-methoxy)-1,3-benzodioxol farblose Blättchen, die bei 158 - 161° schmelzen.

B e i s p i e l 29 :

5 g Natriumsalz von 2,2-Dimethyl-4-hydroxy-5,7-dibrom-1,3-benzodioxol werden nach Zusatz von 0,4 g Benzyltriäthylammoniumchlorid in 10 ml Butylbromid bei 100° 2 Stunden gerührt. Der Eindampfrückstand der Reaktionsmischung wird dann in Essigester aufgenommen, die Lösung mit 1n NaOH und mit Wasser gewaschen und danach eingedampft. Das zurückbleibende gelbliche Öl besteht aus 2,2-Dimethyl-4-butoxy-5,7-dibrom-1,3-benzodioxol, das einen Brechungsindex $n_D^{23}$ = 1,5468 hat und auf Kieselgel 60 F 254 mit dem Laufmittel: Chloroform/Methanol (9 : 1) einen Rf-Wert von 0,82 zeigt.

B e i s p i e l 30 :

Eine Lösung von 10 g des gemäß Beispiel 26 erhältlichen 2,2-Dimethyl-4-(2',3'-epoxy-propoxy)-5,7-dijod-1,3-benzodioxols in 50 ml Äthanol wird mit 2,2 g 2-Amino-2-methyl-1,3-propandiol unter Rückfluß gekocht, bis die Umsetzung gemäß Dünnschicht-Chromatogramm (vgl. Beispiel 18) beendet ist. Dann wird der Eindampfrückstand in Essigester aufgenommen, die Lösung zunächst mit Wasser und dann mit 2n HCl ausgeschüttelt, wobei ein ausfallendes Öl durch Wasserzusatz wieder in Lösung gebracht wird. Die saure wässerige Lösung wird mit Aktivkohle behandelt, filtriert, mit NaOH alkalisch gemacht und mit Chloroform ausgeschüttelt. Die mit $Na_2SO_4$ sicc. getrocknete Chloroformlösung hinterläßt nach dem Eindampfen ein Öl, das nach Auflösen in Essigester und Versetzen mit alkoholischer HCl ein kristallines Produkt ergibt. Dieses schmilzt nach dem Umkristallisieren aus Isopropanol oder Acetonitril bei 205 - 208° und besteht aus dem Hydrochlorid von 2,2-Dimethyl-4-[3'-(α,α-di-hydroxymethyl-äthylamino)-2'-hydroxy-propoxy]-5,7-dijod-1,3-benzodioxol.

B e i s p i e l 31 :

Eine Lösung von 10 g 2,2-Dimethyl-4-hydroxy-1,3-benzodioxol in 20 ml Methanol wird unter Rühren tropfenweise mit 10,83 g einer 30 %igen methanolischen $NaOCH_3$-Lösung versetzt und dann eingedampft. Der Rückstand wird in 15 ml Chloracetaldehyd-dimethylacetal aufgenommen und die Mischung nach Zusatz von 1 g Tetrabutylammoniumbromid mehrere Tage unter Rückfluß erhitzt, bis die Umsetzung gemäß Dünnschicht-Chromatogramm (Schicht: Kieselgel 60 F 254, Laufmittel: Cyclohexan/Aceton = 3 : 1) nahezu beendet ist.

Die Lösung des Eindampfrückstandes in Essigester wird mit verdünnter NaOH gewaschen, über $Na_2SO_4$ sicc. getrocknet, nach Zusatz von Aktivkohle filtriert und eingedampft. Das so erhaltene Öl wird destilliert (Kp.: 155 - 157°/12 Torr) und besteht aus 2,2-Dimethyl-4-(2',2'-dimethoxy-äthoxy)-1,3-benzodioxol. Am Dünnschicht-Chromatogramm (s.o.) zeigt

es einen Rf-Wert von 0,44, das Ausgangsprodukt einen von 0,30. Der Brechungsindex des erhaltenen Dimethylacetals: $n_D^{24}$ = 1,4971.

B e i s p i e l 32:

Werden gemäß Beispiel 30 anstelle von 2-Amino-2-methyl-1,3-propandiol 7,6 g 2-Amino-1,3-propandiol (Serinol) eingesetzt, dann erhält man bei ansonsten gleicher Verfahrensweise das Hydrochlorid von 2,2-Dimethyl-4-[3'-(1",3"-dihydroxyisopropylamino)-2'-hydroxy-propoxy]-5,7-dijod-1,3-benzodioxol, das aus Acetonitril/Methanol umkristallisiert wird und bei 190 - 195° schmilzt.

B e i s p i e l 33 :

Eine Suspension von 10 g des gemäß Beispiel 18 erhältlichen 2,2-Dimethyl-4-(carboxy-methoxy)-1,3-benzodioxols in 150 ml $CH_2Cl_2$ wird unter Rühren mit 3,3 g Acetonoxim versetzt und danach tropfenweise mit einer Lösung von 9,2 g N,N-Dicyclohexyl-carbodiimid in 80 ml $CH_2Cl_2$. Nach 30 Minuten ist die Umsetzung laut Dünnschicht-Chromatogramm (Schicht: Kieselgel 60 F 254, Laufmittel: Cyclohexan/Aceton = 1 : 1) beendet. Der Eindampfrückstand wird in Isopropyläther aufgenommen, der über Nacht ausgefallene Niederschlag abgesaugt und das Filtrat eingedampft. Der kristalline Rückstand wird aus Isopropyläther umkristallisiert. Das so erhaltene Aceton-O-(2,2-dimethyl-1,3-benzodioxol-4-oxy-acetyl)-oxim schmilzt bei 73 - 75°.

Die im folgenden angegebenen neuen Pyrogallol-äther der allgemeinen Formel

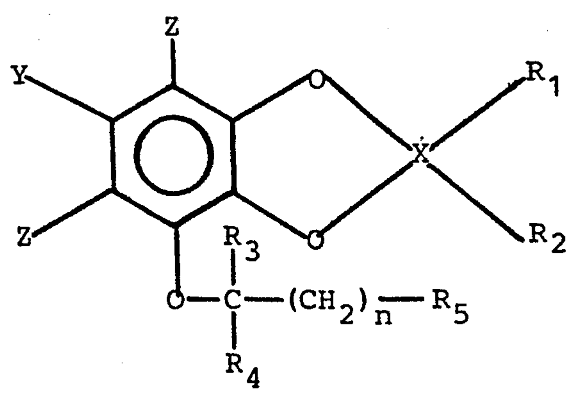 ,

wurden, wie in den Beispielen 17 bis 33 beschrieben, erhalten:

| X | Y | Z | n | $R_5$ | Fp°C |
|---|---|---|---|---|---|
| $R_1$, $R_2$ = $CH_3$ | | | $R_3$, $R_4$ = H | | |
| Br | H | Br | O | COOH | 175-178 |
| J | H | J | O | COOH | 198-200 |
| Cl | H | H | O | COOH | 118-120 |
| $NO_2$ | H | H | O | COOH | 123-125 |
| H | H | $NO_2$ | O | COOH | 165-167 |
| H | H | H | O | 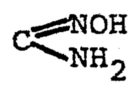 | 130-134 |
| H | H | H | O | 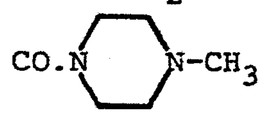 | 106-108 |
| H | H | H | O | $CHOH.CH_2OH$ | 68-70 |
| Cl | H | H | O | 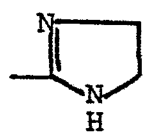 . HCl | 195-205 (Zers.) |
| $R_1$, $R_2$, $R_3$, $R_4$ = $CH_3$ | | | | | |
| Br | H | Br | O | COOH | 161-164 |
| J | H | J | O | COOH | 190-193 |

P a t e n t a n s p r ü c h e :

1. Neue Pyrogallolderivate der allgemeinen Formel

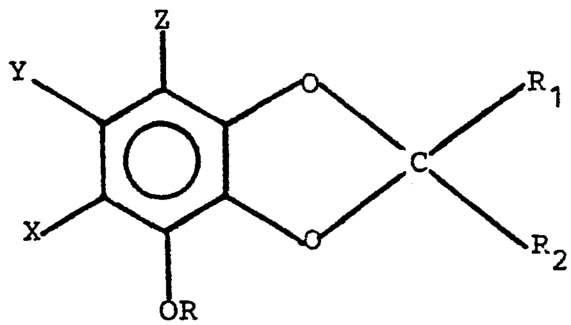

, (I)

worin $R_1$ und $R_2$ gleich oder verschieden sein können und Wasserstoff oder nied.Alkyl bedeuten, und R

(i) Wasserstoff oder

(ii) $-\overset{R_3}{\underset{R_4}{C}}-(CH_2)_n-R_5$ darstellt, wobei

(i) wenn R Wasserstoff bedeutet, X, Y und Z Wasserstoff, Halogen oder Nitro bedeuten, mit der Maßgabe, daß nicht alle drei gleichzeitig Wasserstoff darstellen, und mit der weiteren Maßgabe, daß, wenn $R_1$, $R_2$, X und Z Wasserstoff bedeuten, Y keine $NO_2$-Gruppe sein kann; und

(ii) a) wenn R die Gruppe $-\overset{R_3}{\underset{R_4}{C}}-(CH_2)_n-R_5$ bedeutet, $R_3$ und $R_4$ gleich oder verschieden sind und Wasserstoff oder nied.Alkyl bedeuten, X, Y und Z ebenfalls gleich oder verschieden sind und Wasserstoff, Halogen oder Nitro bedeuten, n für Null, 1 oder 2 steht, $R_5$ Wasserstoff, Oxiranyl, Mono- oder Dihydroxyalkyl, N-(Dihydroxyalkyl)-amino-hydroxyalkyl, Carboxy, Carbalkoxy, Carbamyl, N-Alkylcarbamyl, N,N-Dialkylcarbamyl, Cyan,

$\Delta^2$-Imidazolin-2-yl, Amidoxim, Oximester oder Dialkylacetal darstellt, wobei der Aminrest der Carbamylgruppe auch ein gesättigter Heteroring mit insgesamt zwei Heteroatomen sein kann; oder

b) X Wasserstoff oder Allyl ist, Y, Z, $R_3$ und $R_4$ Wasserstoff sind und n Null ist, $R_5$ die Gruppe

$-CHOH-CH_2-N<^{R_6}_{R_7}$ bedeutet, worin $R_6$ Wasserstoff oder nied.Alkyl sein kann oder zusammen mit $R_7$ einen fünf- oder sechsgliedrigen gesättigten heterocyclischen Ring bildet, der höchstens zwei Heteroatome enthält, $R_7$ Alkyl, Alkenyl, Alkinyl, Alkoxyalkyl, Cycloalkyl, Dialkylaminoalkyl, Aryl, Aralkyl, Heteroalkyl, Carbamoylalkyl oder einen fünf- oder sechsgliedrigen und gegebenenfalls kondensierten heterocyclischen Rest mit höchstens drei Heteroatomen bedeutet,

sowie deren Salze mit anorganischen oder organischen Säuren oder Basen.

2. 2,2-Dimethyl-4-(2'-hydroxy-3'-tert.butylaminopropoxy)-1,3-benzodioxol bzw. dessen Salz mit einer pharmazeutisch annehmbaren Säure, vorzugsweise das Hydrochlorid.

3. Verwendung von neuen kernsubstituierten Pyrogallol-Derivaten der allgemeinen Formel

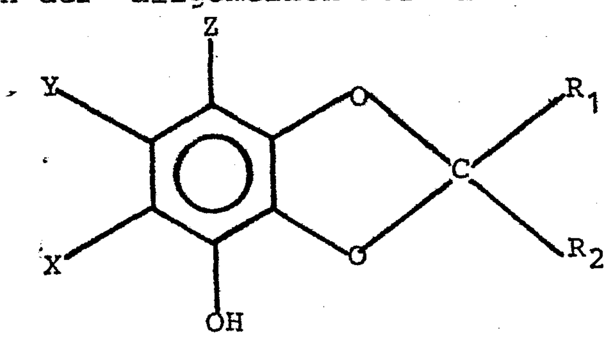

, (Ia)

worin $R_1$, $R_2$, X, Y und Z die in Anspruch 1 unter (i) angegebene Bedeutung haben, sowie deren Salzen mit Basen als Agrochemikalien oder als Rohstoffe zur Herstellung von solchen.

4. Verwendung von neuen kernsubstituierten Pyrogallol-Derivaten der in Anspruch 3 angegebenen allgemeinen Formel

(Ia), worin $R_1$, $R_2$, X, Y und Z die dort angegebene Bedeutung haben, sowie deren Salzen mit Basen als Rohstoffe zur Herstellung von Arzneimitteln, Röntgenkontrastmitteln oder Fotosensibilisatoren.

5. Verwendung von neuen kernsubstituierten Pyrogallol-Derivaten der allgemeinen Formel

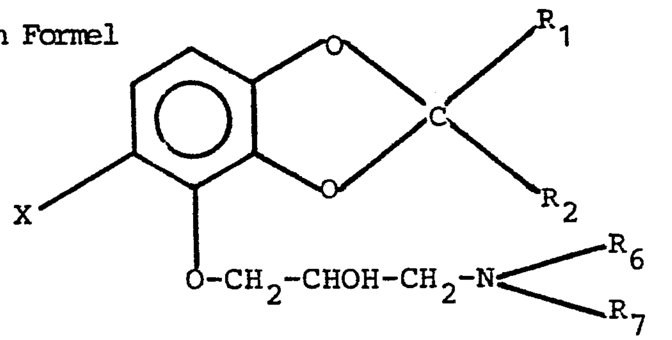

, (Ib)

worin $R_1$, $R_2$, X, $R_6$ und $R_7$ die in Anspruch 1 unter (ii) b) angegebene Bedeutung haben, gegebenenfalls in Form der optisch aktiven Isomere, als ß-Blocker und Analgetika.

6. Verwendung von neuen kernsubstituierten Pyrogallol-Derivaten der in Anspruch 5 angegebenen Formel (Ib) in Form von Salzen mit Ionenaustauschern als ß-Blocker und Analgetika.

7. Verfahren zur Herstellung der neuen Pyrogallolderivate der allgemeinen Formel (Ia), dadurch gekennzeichnet, daß man ein 4-Hydroxy-1,3-benzodioxol der allgemeinen Formel

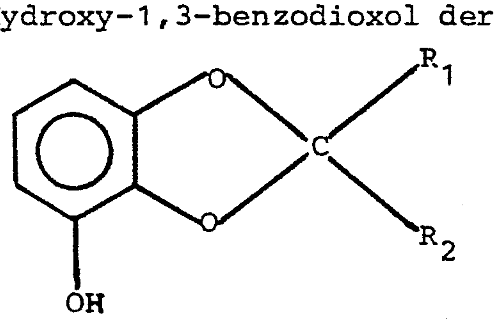

, (II)

worin $R_1$ und $R_2$ die in Anspruch 1 angegebene Bedeutung haben, vorzugsweise in einem inerten Lösungsmittel oder Lösungsmittelgemisch, in ein- oder zweistufiger Reaktion, gegebenenfalls unter Verwendung von säurebindenden Zusätzen und/oder Katalysatoren, mit Halogenierungs- und/oder Nitrierungsmitteln behandelt.

8. Verfahren zur Herstellung der neuen Pyrogalloläther der allgemeinen Formel

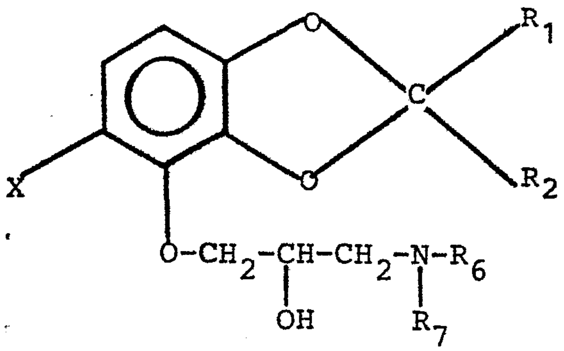

, (Ib)

worin X, $R_1$, $R_2$, $R_6$ und $R_7$ die in Anspruch 1 unter (ii) b) angegebene Bedeutung haben, sowie deren Salzen und optisch aktiven Isomeren, dadurch gekennzeichnet, daß man ein 4-Hydroxy-1,3-benzodioxol der allgemeinen Formel

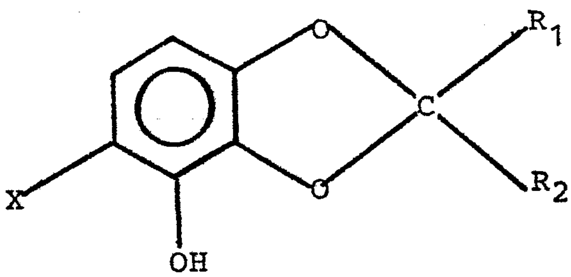

, (III)

worin $R_1$, $R_2$ und X die obige Bedeutung haben, oder ein Alkalisalz hievon mit einem Epihalohydrin umsetzt, das dabei erhaltene Produkt der allgemeinen Formel

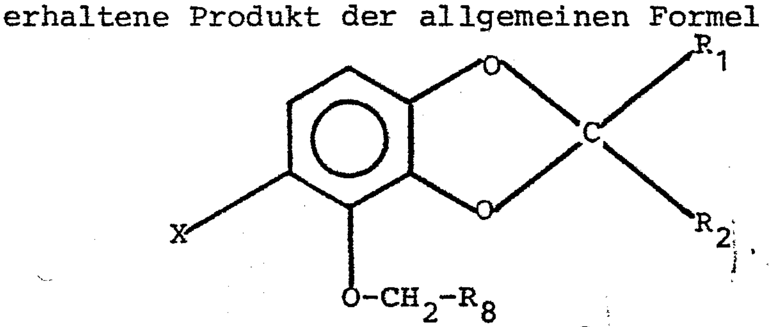

, (IV)

worin $R_8$ $-CHOH.CH_2Q$ oder 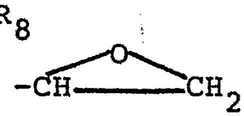 bedeutet, wobei Q ein Halogenatom darstellt, mit einem Amin der allgemeinen Formel

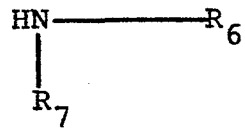

, (V)

worin $R_6$ und $R_7$ die oben angegebene Bedeutung haben, behandelt und das dabei erhaltene Derivat der allgemeinen Formel (Ib) gewünschtenfalls in die optischen Antipoden spaltet

und/oder gegebenenfalls mit einer physiologisch verträglichen Säure oder Base in ein Salz überführt.

9. Verfahren nach Anspruch 8, dadurch gekennzeichnet, daß man als physiologisch verträgliche Säure zur Salzbildung einen Ionenaustauscher einsetzt.

10. Verfahren zur Herstellung der neuen Derivate von Pyrogallol-äthern der allgemeinen Formel

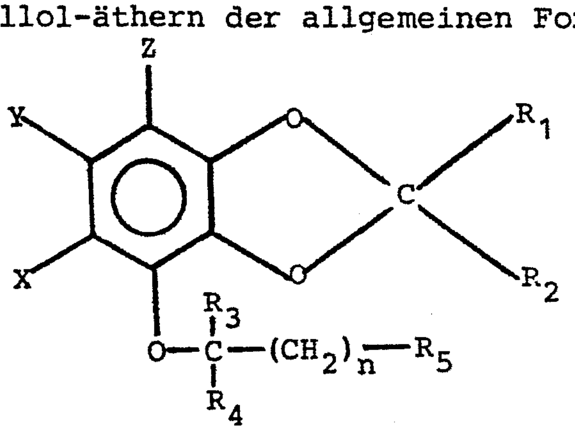

, (Ic)

worin $R_1$, $R_2$, $R_3$, $R_4$, $R_5$, X, Y, Z und n die in Anspruch 1 unter (ii) a) angegebene Bedeutung haben, dadurch gekennzeichnet, daß man ein Pyrogallol-Derivat der allgemeinen Formel

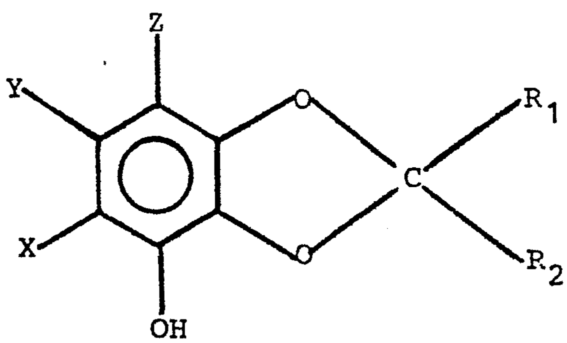

, (VI)

worin $R_1$, $R_2$, X, Y und Z die obige Bedeutung haben, oder ein Phenolat hievon, vorzugsweise in inerten Lösungsmitteln oder Lösungsmittelgemischen, mit einer Verbindung der allgemeinen Formel

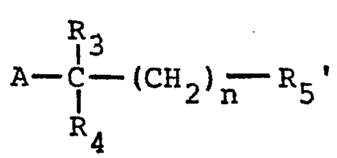

, (VII)

worin $R_3$, $R_4$ und n obige Bedeutung haben, $R_5'$ die gleiche Bedeutung wie $R_5$ hat oder eine CH.CN-Gruppe bedeutet und A Halogen, einen Sulfonyloxyrest oder für den Fall, daß $R_3$

und $R_4$ Wasserstoff, n = Null und $R_5'$ eine CH.CN-Gruppe darstellen, eine Doppelbindung mit dem benachbarten Kohlenstoffatom bedeutet, umsetzt, gegebenenfalls in einem erhaltenen Derivat der allgemeinen Formel (Ic) eine Gruppe $R_5$ in eine andere Gruppe $R_5$ überführt und, für den Fall, daß X und/oder Y und/oder Z Wasserstoff bedeuten, die erhaltene Verbindung der allgemeinen Formel (Ic) gewünschtenfalls mit Halogenierungs- und/oder Nitrierungsmitteln behandelt.